# EUROPEAN PATENT SPECIFICATION

(11) **EP 4 015 526 B1**
(45) Date of publication and mention of the grant of the patent: **30.10.2024**
(21) Application number: 20868135.3
(22) Date of filing: 24.09.2020
(51) Int. Cl.: C07K 14/54, C12N 15/24, C12N 15/63

(54) **RECOMBINANT INTERLEUKIN-15 ANALOG**
REKOMBINANTES INTERLEUKIN-15-ANALOG
ANALOGUE DE L'INTERLEUKINE-15 RECOMBINÉ

(30) Priority: 25.09.2019 CN 201910910158
(43) Date of publication of application: 22.06.2022
(73) Proprietor: Leto Laboratories Co., Ltd, Changping District Huilongguan Town Beijing 100026 (CN)
(72) Inventor: ZHAO, Yao, Beijing 100026 (CN); ZHANG, Jianjun, Beijing 100026 (CN); ZHU, Xiaoting, Beijing 100026 (CN); XU, Yanling, Beijing 100026 (CN); PENG, Lujia, Beijing 100026 (CN); WANG, Jishu, Beijing 100026 (CN); ZHANG, Wei, Beijing 100026 (CN)
(74) Representative: Sun, Yiming
(86) International application number: PCT/CN2020/117279
(87) International publication number: WO 2021/057826

(56) References cited:
- WO-A2-2012/110878
- CN-A- 1 921 879
- CN-A- 1 921 879
- CN-A- 108 147 990
- CN-A- 108 147 990
- DATABASE Geneseq [online] 6 November 2014 (2014-11-06), "Human IL-15 variant T95X, SEQ ID 8.", XP002807948, retrieved from EBI accession no. GSP:BBN47332 Database accession no. BBN47332
- DATABASE Geneseq [online] 11 October 2012 (2012-10-11), "Human IL15-human alpha-synuclein SP fusion protein sequence SEQ:8.", XP002807949, retrieved from EBI accession no. GSP:AZZ02884 Database accession no. AZZ02884
- LEE SOO-HYEON ET AL: "Generation of recombinant canine interleukin-15 and evaluation of its effects on the proliferation and function of canine NK cells", VETERINARY IMMUNOLOGY AND IMMUNOPATHOLOGY, vol. 165, no. 1-2, 1 May 2015 (2015-05-01), AMSTERDAM, NL, pages 1 - 13, XP055980332, ISSN: 0165-2427, DOI: 10.1016/j.vetimm.2015.04.002
- HONGYUAN MAO ET AL: "Sortase-Mediated Protein Ligation: A New Method for Protein Engineering", JOURNAL OF THE AMERICAN CHEMICAL SOCIETY, vol. 126, no. 9, 1 March 2004 (2004-03-01), pages 2670 - 2671, XP055060278, ISSN: 0002-7863, DOI: 10.1021/ja039915e
- LIM SUNG IN ET AL: "Site-specific fatty acid-conjugation to prolong protein half-life in vivo", JOURNAL OF CONTROLLED RELEASE, ELSEVIER, AMSTERDAM, NL, vol. 170, no. 2, 2 June 2013 (2013-06-02), pages 219 - 225, XP028676591, ISSN: 0168-3659, DOI: 10.1016/J.JCONREL.2013.05.023

## Description

### TECHNICAL FIELD

The present invention belongs to the field of molecular biology, and particularly relates to a recombinant interleukin-15 analog and expression thereof.

### BACKGROUND ART

Interleukin-15 (IL-15) is a cytokine having a size of about 12 to 14 kD. The mature peptide of natural human interleukin-15 contains 114 amino acids, including 4 cysteine residues. Two pairs of intramolecular disulfide bonds respectively formed by the connection of Cys35 and Cys85 and the connection of Cys42 and Cys88 play an important role in maintaining the spatial conformation and biological activity of IL-15.

Similar to most cytokines, IL-15 plays a role in normal immune responses, such as promoting the development of T cells, B cells and natural killer (NK) cells. IL-15 and IL-2 share the same β chain and γ chain receptors, thus their biological activities are very similar. Due to the different α chain receptors, IL-2 can activate Treg cells and induce Teff and NK cell apoptosis (AICD), thus the clinical application of IL-2 is greatly limited. In contrast, IL-15 of the same family does not have the functions of Treg activation and AICD, thus IL-15 has great therapeutic potential.

The α subunit of the IL-15 receptor has a high affinity for IL-15. Under physiological conditions, IL-15 mostly forms a complex (IL-15-Rα) with the α subunit, which enhances the affinity of IL-15 for the β chain subunit and the γ chain subunit of the receptor, and activates T cells and NK cells. Thereby, companies such as ALTOR and Hengrui have utilized the characteristics of the α subunit to formed a complex with IL-15 and α subunit (or its part) through fusion or non-fusion methods, which has shown good biological potency and stability in animal experiments.

The α subunit of IL-15 receptor is expressed on the surface of myeloid cells, including macrophages, antigen-presenting cells, NK cells and T cells. It also plays an important role in anti-tumor *in vivo.* Therefore, the IL-15-Rα complex is independent of the α subunit *in vivo.* Although the stability of IL-15 monomer is improved, it has a subtle functional difference from native IL-15, which may reveal essential differences in antitumor *in vivo.*

Human IL-15 variant T95X was described (Geneseq, 6 November 2014, EBI accession no. GSP:BBN47332, Database accession no. BBN47332). Human IL 15-human alpha-synuclein SP fusion protein sequence is described (Geneseq, 11 October 2012, EBI accession no. GSP:AZZ02884, and WO 2012/110878 A2). Lee et al. generated an rclL-15 protein consisting of Axn-49-Ser-162 with a C-terminal His tag and examiner its functions ex vivo in term of the proliferation and antitumor effects on canine noon-B, non-T, large granular natural killer (NK) cells (Lee et al., Veterinary immunology and Immunopathology, vol. 165, no. 1-2, 2015, 1-13).

IL-15 has higher safety and activity as compared to IL-2. However, as a protein drug, natural wild-type IL-15 has significant drug development bottlenecks, including low expression levels in prokaryotes and eukaryotes, difficult purification and short half-life, making it difficult to be industrialized. Therefore, it is necessary to study the exogenous expression of IL-15 to obtain high production efficiency. In addition, the N-terminal or C-terminal of IL-15 analog obtained by *in vitro* renaturation can be coupled to the fatty acid chain of human serum albumin binder, so as to achieve a long-term efficacy, which has certain significance for the treatment of tumors.

### SUMMARY OF INVENTION

In view of the low production efficiency of IL-15 in the prior art, the present disclosure provides an IL-15 analog with high expression level and relatively simple purification process. Further, the present disclosure provides a conjugate of the IL-15 analog, thus as to improve the half-life of IL-15 and the long-term efficacy thereof. The present invention is defined in the appended claims.

The present inventors have found that the expression of IL-15 in *Escherichia Coli* could be promoted by adding some amino acids to the C-terminal of IL-15, and that positively charged amino acids could significantly enhance the expression of IL-15. The present IL-15 analog is highly expressed in *Escherichia Coli,* and the expression level is about 20 or even more fold higher than that of IL-15 without extra amino acids at the C-terminal. All or most of the present IL-15 analog retains the amino acid sequence of the natural wild-type IL-15, and there is no significant difference in cell activity *in vitro,* which lays a foundation for the industrialization of IL-15 protein drugs.

In the present disclosure, a fatty acid chain is linked with the IL-15 analog through *in vitro* coupling to form a coupling product of IL-15 analog-fatty acid chain. Since the fatty acid chain is a ligand of albumin and can bind to albumin in blood, the IL-15 analog-fatty acid chain coupling product entering the body will form an IL-15 analog-fatty acid chain-albumin complex. On one hand, it can increase the molecular weight to escape from the renal filtration. On the other hand, hydrolysis by intracellular lysosomes can be avoided through the binding of albumin to FcRn which mediates recycling pathway as a protection mechanism, thereby achieving a long-acting mechanism of IL-15 analog-fatty acid chain coupling product. Herein, the conjugate of the present IL-15 analog is not in the form of a drug that is co-expressed with the IL-15 receptor α subunit to form a complex as used by most domestic and foreign companies. Instead, it retains the same cellular biological pattern as native IL-15, that is, it completely retains its binding to the IL15Rα receptor to participate in signal transduction and to function *in vivo.*

### DESCRIPTION OF THE DRAWINGS

FIG. 1 shows the SDS-PAGE electrophoretogram of wild-type IL-15 expressed in *Escherichia Coli* according to a comparative example of the present disclosure.

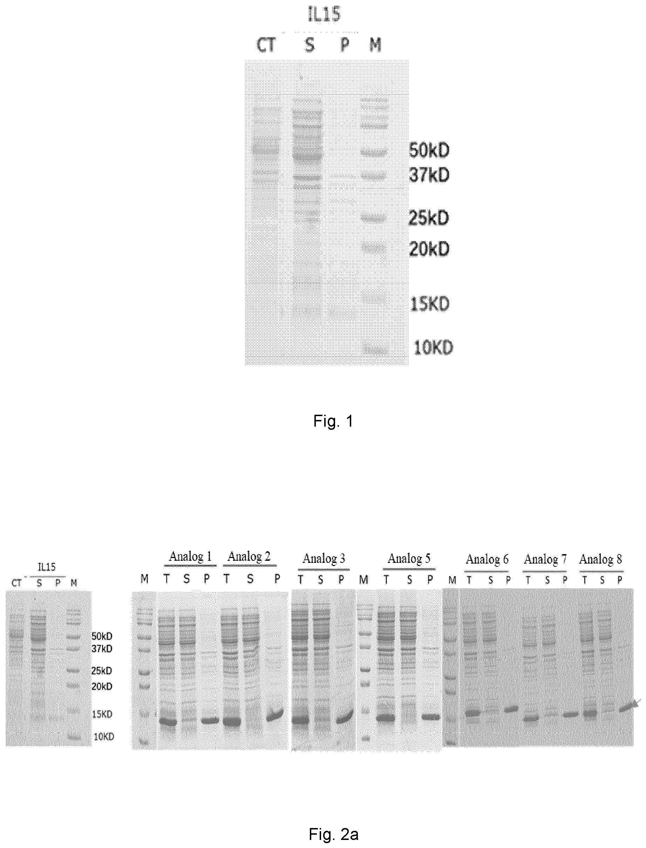

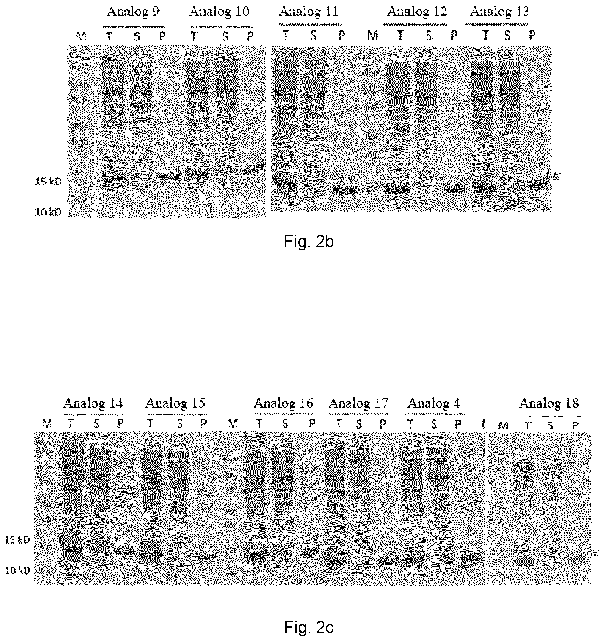

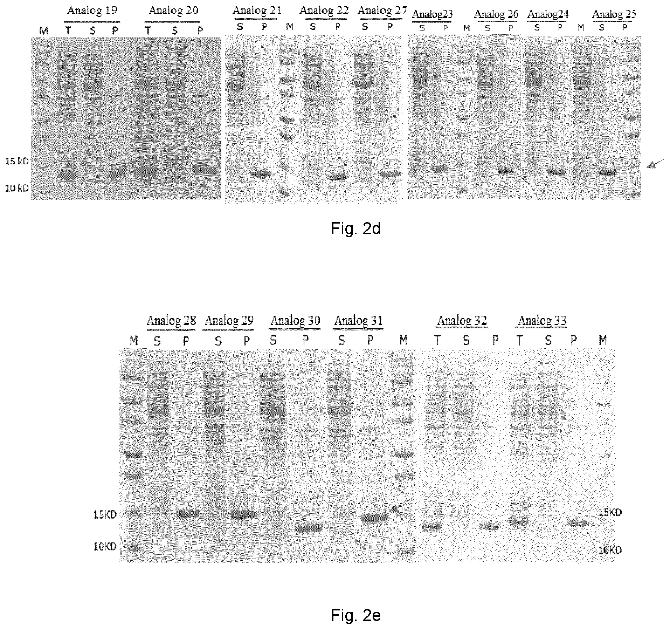

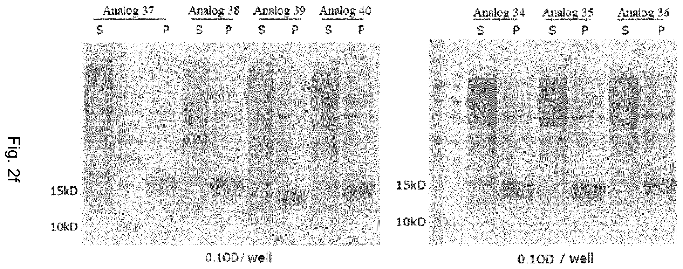

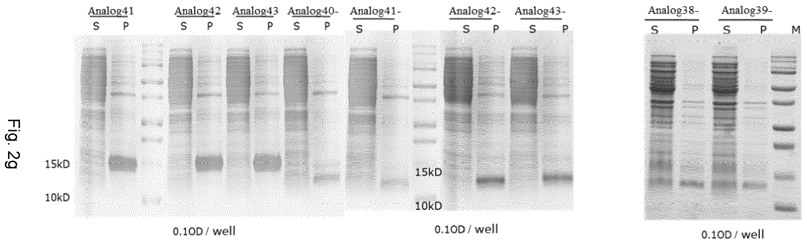

Fig. 2 shows the SDS-PAGE electrophoretograms of IL-15 analogs expressed in *Escherichia Coli* according to an Example of the present disclosure, wherein Fig. 2a shows the SDS-PAGE electrophoretograms of IL-15 analogs 1 to 3 and 5 to 8, Fig. 2b shows the SDS-PAGE electrophoretograms of IL-15 analogs 9 to 13, Fig. 2c shows the SDS-PAGE electrophoretograms of IL-15 analogs 4 and 14 to 18, Fig. 2d shows the SDS-PAGE electrophoretograms of IL-15 analogs 19 to 27, Fig. 2e shows the SDS-PAGE electrophoretograms of IL-15 analogs 28 to 33, and Figs. 2f and 2g show the SDS-PAGE electrophoretograms of IL-15 analogs 34 to 43, with analogs 38- to 43-representing that no specific amino acid were added to the C-terminal.
Fig. 3 shows comparison results of the expression levels of IL-15 and IL-15 analogs 1 to 33 (unit: mg/mL/10OD).
Fig. 4 shows SDS-PAGE electrophoretograms of renatured and purified IL-15 and IL-15 analogs 11, 18, 21 and 28, wherein the results of reduced SDS-PAGE are displayed on the left side of Marker, with the reducing agent 2-mercaptoethanol added to the electrophoresis loading buffer, and the results of non-reduced SDS-PAGE are displayed on the right side of Marker, without the reducing agent 2-mercaptoethanol added to the electrophoresis loading buffer.
FIG. 5 shows the chromatogram of the purity of the conjugate of IL-15 analog in Example 3, which was determined by RP-UPLC (purity>95%).
FIG. 6 shows the molecular weight of the conjugate of IL-15 analog in Example 3, which was determined by LC-MS.
FIG. 7 shows the molecular weight of the conjugate of IL-15 analog in Example 4, which was determined by LC-MS.
FIG. 8 shows the antitumor effects of IL-15 Analog 21 and conjugate of IL-15 analog in mice in Example 7.

### DETAILED DESCRIPTION

Hereinafter, the present invention will be further described in conjunction with examples.

In the following examples, the experimental methods without special instructions were usually carried out in accordance with conventional conditions or in accordance with the conditions recommended by the manufacturer. See, for example, Sambrook et al, Molecular Cloning: A Laboratory Manual (New York: Cold Spring Harbor Laboratory Press, 1989). Unless otherwise specified, the reagents used are commercially available or publicly available reagents.

In particular embodiments according to the present disclosure, the positively charged amino acids were leucine (Lys, K), arginine (Arg, R) and histidine (His, H).

As the basis for modification, the amino acid sequence of IL-15 was selected from the group of:
1) the amino acid sequence shown in SEQ ID NO. 1;
2) an amino acid sequence derived from the amino acid sequence shown in SEQ ID NO. 1 through substitution, deletion or addition of one or more amino acids; and
3) an amino acid being at least 80%, 85%, 90%, 91%, 92%, 93%, 94%, 95%, 96%, 97%, 98% or 99% identical to the amino acid sequence shown in SEQ ID NO. 1.

In particular embodiments according to the present disclosure, the expression of IL-15, especially in prokaryotic expression systems, could be enhanced by adding one or more amino acids, especially positively charged amino acids, to the C-terminal of IL-15.

In some particular embodiments, the amino acid sequence of the IL-15 analog could be represented as the following general formula (that is, the amino acid sequence Xₐ-Y_{b}-Z_{c} was added to the C-terminal of IL-15):

IL-15-Xₐ-Y_{b}-Z_{c}

wherein X, Y and Z each represented an amino acid sequence added at the C-terminal, and a, b and c each represented the number of the amino acids; and
wherein X and Z each were any amino acid or a combination of any amino acids, and a and c each were 0 to 20; and
wherein Y was a positively charged amino acid or a combination of any positively charged amino acids, or a combination of a positively charged amino acid and any other amino acids, and b was 1 to 7.

In some particular embodiments, the amino acid sequence of the IL-15 analog could be represented as the following general formula (that is, a linker sequence and the amino acid sequence Xₐ-Y_{b}-Z_{c} were added to the C-terminal of IL-15):

IL-15-linker-Xₐ-Y_{b}-Z_{c}

wherein X, Y and Z each represented an amino acid sequence added at the C-terminal, and a, b and c each represented the number of the amino acids; and
wherein X and Z each were any amino acid or a combination of any amino acids, and a and c each were 0 to 20; and
wherein Y was a positively charged amino acid or a combination of any positively charged amino acids, or a combination of a positively charged amino acid and any other amino acids, and b was 1 to 7.

In some optional particular embodiments, the linker could be (GGGGS)ₙ, (GS)ₙ or (GAPQ)ₙ, with n being 1 to 5.

In some particular embodiments, in the above general formula of the amino acid sequence of IL-15 analogs, Xₐ comprised LPBTG, wherein B was any amino acid and the linker was (GS)ₙ, with n being 0 to 10. In a particular embodiment, the IL-15 analog was obtained by adding a sequence comprising -GS-LPETG to the terminal of the amino acid sequence of IL-15.

In some particular embodiments, the IL-15 analogs were coupled with fatty acid chains *in vitro* to improve the long-term efficacy of the IL-15 analogs. The way of *in vitro* coupling of fatty acid chains could be selected from the group of:
1) coupling with the amino group at the N-terminal, for example, through an aldehyde group or a succinimide ester;
2) site-directed coupling with the introduced free cysteine residue (for example, introducing a free cysteine residue at the C-terminal of the IL-15 analog), through a maleimide group or a halogenated group; and
3) utilizing an enzymatic reaction, for example, the enzyme Sortase A could utilize the small peptide LPETG at the C-terminal of IL-15 to couple the IL-15 with the fatty acid chain with GGG at the N-terminal(see M. L. Bentley et al., J. Biol. Chem. 2008, 283: 14762-14771). The fatty acid chains could comprise the structure as shown in Table 1.

**Table 1: The structure of fatty acid chains**

| Name | Fatty acid chain | Reaction with IL-15 |
|---|---|---|
| 81636 6 | GGG-PEG2-Lys-(CH₂)₁₆-COOH | (IL-15)-LPET-816366 |
| 82347 9 | GGG-PEG4-PEG4-PEG4-Lys-(CH₂)₁₇-COOH | (IL-15)-LPET-823479 |
| 82348 0 | GGG-PEG4-γ-Glu-γ-Glu-Lys-(CH₂)₁₇-COOH | (IL-15)-LPET-823480 |
| 81838 9 | HOOC-(CH₂)₁₆-γ-Glu-γ-Glu-Lys-GGG | (IL-15)-LPET-818389 |
| 81754 9 | HOOC-(CH₂)₁₆-CONH-γ-Glu-γ-Glu-PEG2-Lys-Br | (IL-15)-Cysteine-817549 |
| 82384 8 | GGG-γ-Glu-C2DA-2OEG-γ-Glu-(CH₂)₁₇-COOH | (IL-15)-LPET-823848 |
| 82385 3 | GGG-γ-Glu-C2DA-2OEG-γ-Glu-(CH₂)₁₉-COOH | (IL-15)-LPET-823853 |
| 82385 4 | GGG-OEG-C2DA-2OEG-γ-Glu-(CH₂)₁₉-COOH | (IL-15)-LPET-823854 |
| 82385 6 | GGG-OEG-C2DA-2OEG-γ-Glu-Trx-(CH₂)₁₉-COOH | (IL-15)-LPET-823856 |
| 82004 4 | NHS-PEG2-PEG2-γ-Glu-(CH₂)₁₇-COOH | 820044-(IL-15) |
| 82004 5 | CHO-PEG2-PEG2-γ-Glu-(CH₂)₁₇-COOH | 820045-(IL-15) |
| 82385 5 | Mal-C2DA-2OEG-γ-Glu-Tn-(CH₂)₁₉-COOH | (IL-15)-Cysteine-823855 |

| | | |
|---|---|---|
| *In the above table, LPET represents that the amino acid sequence added to the C-terminal of IL-15 includes LPET. | | |

### Example 1: Expression of wild-type IL-15 and IL-15 analogs in Escherichia Coli

### 1.1 Construction of expression vector

The wild-type IL-15 nucleotide sequence was synthesized by Sangon Biotech (Shanghai).

### (1) Design of primers

The sequence of the C-terminal of IL-15 was altered by introducing a base sequence of different amino acids to be added into the reverse primer. The amino acid sequence, the nucleotide sequence and the primer sequence (used in the construction of IL-15 analogs) of the wild-type IL-15 and constructed IL-15 analogs are shown in Table 2.

**Table 2: Amino acid sequence, nucleotide sequence and primer sequence (used in the construction of IL-15 analogs) of the wild-type IL-15 and IL-15 analogs**

| | Sequence | SEQ ID NO. |
|---|---|---|
| IL-15 | | 1 |
| | ATGAACTGGGTGAACGTTATCAGCGACCTGAAGAAAATCG AGGATCTGATTCAGAGCATGCACATTGACGCGACCCTGTA CACCGAAAGCGATGTGCACCCGAGCTGCAAGGTTACCGC GATGAAATGCTTCCTGCTGGAGCTGCAAGTGATCAGCCTG GAAAGCGGTGACGCGAGCATTCACGATACCGTTGAGAAC CTGATCATTCTGGCGAACAACAGCCTGAGCAGCAACGGTA ACGTGACCGAGAGCGGCTGCAAGGAATGCGAGGAACTGG AGGAAAAGAACATCAAAGAATTCCTGCAGAGCTTTGTGCA CATCGTTCAAATGTTTATTAACACCAGC（SEQ ID NO. 2） | 2 |
| Analo g 1 | IL-15-(GS)₃-HHHHHH | |
| | I L- 1 5-ggttctggttctggttctcaccaccaccaccaccac | |
| | Forward primer for Analog 1: | 23 |
| | taagaaggagatatacatatgAACTGGGTGAACGTTATCAGCG | |
| | Reverse primer 1 for Analog 1 : | 24 |
| | gtggtggtggtggtgagaaccagaaccagaaccGCTGGTGTTAATAAAC ATTTGAACG | |
| | Reverse primer 2 for Analog 1 : | 25 |
| | gtggtggtggtggtgctcgagTTAGTGGTGGTGGTGGTGAGAA CC | |
| Analo g 2 | IL-15-(GS)₁-HHHHHH | |
| | I L- 1 5-ggttctcaccaccaccaccaccac | |
| | Forward primer for Analog 2: | 26 |
| | taagaaggagatatacatatgAACTGGGTGAACGTTATCAGCG | |
| | Reverse primer 1 for Analog 2: | 27 |
| | gtggtggtggtggtggtgagaaccGCTGGTGTTAATAAACATTTGAAC G | |
| | Reverse primer 2 for Analog 2: | 28 |
| | gtggtggtggtggtgctcgagTTAGTGGTGGTGGTGGTGGTGAG | |
| Analo g 3 | IL-15-PLASTKKR | |
| | IL- 1 5-ccacttgctagcaccaaaaagcgt | |
| | Forward primer for Analog 3: | 29 |
| | taagaaggagatatacatatgAACTGGGTGAACGTTATCAGCG | |
| | Reverse primer 1 for Analog 3: | 30 |
| | acgctttttggtgctagcaagtggGCTGGTGTTAATAAACATTTGAACG | |
| | Reverse primer 2 for Analog 3: | 31 |
| | gtggtggtggtggtgctcgagTTAACGCTTTTTGGTGCTAGCAAG | |
| Analo g 4 | IL-15-LPKSAKKK | |
| | IL-15-cttccaaagtctgctaaaaagaag | |
| | Forward primer for Analog 4: | 32 |
| | taagaaggagatatacatatgAACTGGGTGAACGTTATCAGCG | |
| | Reverse primer 1 for Analog 4: | 33 |
| | cttctttttagcagactttggaagGCTGGTGTTAATAAACATTTGAACG | |
| | Reverse primer 2 for Analog 4: | 34 |
| | gtggtggtggtggtgctcgagTTACTTCTTTTTAGCAGACTTTGGAAG G | |
| Analo g 5 | IL-15-KKKKKKK | |
| | IL-15-aaaaagaagaaaaaaaagaag | |
| | Forward primer for Analog 5: | 35 |
| | taagaaggagatatacatatgAACTGGGTGAACGTTATCAGCG | |
| | Reverse primer 1 for Analog 5: | 36 |
| | cttcttttttttcttctttttGCTGGTGTTAATAAACATTTGAACG | |
| | Reverse primer 2 for Analog 5: | 37 |
| | gtggtggtggtggtgctcgagTTACTTCTTTTTTTTCTTCTTTTTGCTG G | |
| Analo g 6 | IL-15-(GAPQGAPQ)-LVESAHHH | |
| | IL-15-ggtgctccacagggtgctccacagcttgtggaatctgcacaccatcat | |
| | Forward primer for Analog 6: | 38 |
| | taagaaggagatatacatatgAACTGGGTGAACGTTATCAGCG | |
| | Reverse primer 1 for Analog 6: | 39 |
| | attccacaagctgtggagcaccctgtggagcaccGCTGGTGTTAATAAAC ATTTGAACG | |
| | Reverse primer 2 for Analog 6: | 40 |
| | gtggtggtggtggtgctcgagTTAatgatggtgtgcagATTCCACAAGCTGT GGAGCAC | |
| | IL-15-GS-LVSSAHHK | |
| Analo g 7 | IL-15-ggtagccttgtatctagcgctcaccacaaa | |
| | Forward primer for Analog 7: | 41 |
| | taagaaggagatatacatatgAACTGGGTGAACGTTATCAGCG | |
| | Reverse primer 1 for Analog 7: | 42 |
| | tttgtggtgagcgctagatacaaggctaccGCTGGTGTTAATAAACATTT GAACG | |
| | Reverse primer 2 for Analog 7: | 43 |
| | gtggtggtggtggtgctcgagTTATTTGTGGTGAGCGCTAGATACAA | |
| Analo g 8 | IL-15-GS-LIEHHRRK | |
| | IL-15-ggtagccttatcgaacaccaccgtcgcaaa | |
| | Forward primer for Analog 8: | 44 |
| | taagaaggagatatacatatgAACTGGGTGAACGTTATCAGCG | |
| | Reverse primer 1 for Analog 8: | 45 |
| | tttgcgacggtggtgttcgataaggctaccGCTGGTGTTAATAAACATTT GAACG | |
| | Reverse primer 2 for Analog 8: | 46 |
| | gtggtggtggtggtgctcgagTTATTTGCGACGGTGGTGTTCG | |
| Analo g 9 | IL-15-GS-IVEHRKKK | |
| | IL-15-ggtagcattgtagaacaccgtaagaaaaag | |
| | Forward primer for Analog 9: | 47 |
| | taagaaggagatatacatatgAACTGGGTGAACGTTATCAGCG | |
| | Reverse primer 1 for Analog 9: | 48 |
| | ctttttcttacggtgttctacaatgctaccGCTGGTGTTAATAAACATTTGA ACG | |
| | Reverse primer 2 for Analog 9: | 49 |
| | gtggtggtggtggtgctcgagTTACTTTTTCTTACGGTGTTCTACAAT GC | |
| Analo g 10 | IL-15-GS-VPKTGRRR | |
| | IL-15-ggtagcgtaccaaaaactggtcgtcgccgt | |
| | Forward primer for Analog 10: | 50 |
| | taagaaggagatatacatatgAACTGGGTGAACGTTATCAGCG | |
| | Reverse primer 1 for Analog 10: | 51 |
| | acggcgacgaccagtttttggtacgctaccGCTGGTGTTAATAAACATTT GAACG | |
| | Reverse primer 2 for Analog 10: | 52 |
| | gtggtggtggtggtgctcgagTTAACGGCGACGACCAGTTTTT | |
| Analo g 11 | IL-15-GS-LVASGKK | |
| | IL-15-ggtagcctggttgctagcggtaaaaag | |
| | Forward primer for Analog 11: | 53 |
| | taagaaggagatatacatatgAACTGGGTGAACGTTATCAGCG | |
| | Reverse primer 1 for Analog 11 : | 54 |
| | ctttttaccgctagcaaccaggctaccGCTGGTGTTAATAAACATTTGA ACG | |
| | Reverse primer 2 for Analog 11 : | 55 |
| | gtggtggtggtggtgctcgagTTACTTTTTACCGCTAGCAACCAGG | |
| Analo g 12 | IL-15-GS-HRKSGHHH | |
| | IL-15-ggtagccatcgtaaatctggtcaccatcat | |
| | Forward primer for Analog 12: | 56 |
| | taagaaggagatatacatatgAACTGGGTGAACGTTATCAGCG | |
| | Reverse primer 1 for Analog 12: | 57 |
| | atgatggtgaccagatttacgatggctaccGCTGGTGTTAATAAACATTT GAACG | |
| | Reverse primer 2 for Analog 12: | 58 |
| | gtggtggtggtggtgctcgagTTAATGATGGTGACCAGATTTACGAT G | |
| Analo g 13 | IL-15-GS-LPKTGRHK | |
| | IL-15-ggtagccttccaaaaactggtcgtcacaag | |
| | Forward primer for Analog 13: | 59 |
| | taagaaggagatatacatatgAACTGGGTGAACGTTATCAGCG | |
| | Reverse primer 1 for Analog 13: | 60 |
| | cttgtgacgaccagtttttggaaggctaccGCTGGTGTTAATAAACATTTG AACG | |
| | Reverse primer 2 for Analog 13: | 61 |
| | gtggtggtggtggtgctcgagTTACTTGTGACGACCAGTTTTTGGA | |
| Analo g 14 | IL-15-KKKTGRRH | |
| | IL-15-aaaaagaagactggtcgtcgccat | |
| | Forward primer for Analog 14: | 62 |
| | taagaaggagatatacatatgAACTGGGTGAACGTTATCAGCG | |
| | Reverse primer 1 for Analog 14: | 63 |
| | atggcgacgaccagtcttctttttGCTGGTGTTAATAAACATTTGAACG | |
| | Reverse primer 2 for Analog 14: | 64 |
| | gtggtggtggtggtgctcgagTTAATGGCGACGACCAGTCTTCTT | |
| Analo g 15 | IL-15-LPRSGRHK | |
| | IL-15-cttccacgttctggtcgtcataag | |
| | Forward primer for Analog 15: | 65 |
| | taagaaggagatatacatatgAACTGGGTGAACGTTATCAGCG | |
| | Reverse primer 1 for Analog 15: | 66 |
| | cttatgacgaccagaacgtggaagGCTGGTGTTAATAAACATTTGAA CG | |
| | Reverse primer 2 for Analog 15: | 67 |
| | gtggtggtggtggtgctcgagTTACTTATGACGACCAGAACGTGGA | |
| Analo g 16 | IL-15-LVETHHHH | |
| | IL-15-ctggttgaaactcaccatcatcac | |
| | Forward primer for Analog 16: | 68 |
| | taagaaggagatatacatatgAACTGGGTGAACGTTATCAGCG | |
| | Reverse primer 1 for Analog 16: | 69 |
| | gtgatgatggtgagtttcaaccagGCTGGTGTTAATAAACATTTGAAC G | |
| | Reverse primer 2 for Analog 16: | 70 |
| | gtggtggtggtggtgctcgagTTAGTGATGATGGTGAGTTTCAACCA G | |
| Analo g 17 | IL-15-VRPETHHH | |
| | IL-15-gttcgtccagaaactcaccatcat | |
| | Forward primer for Analog 17: | 71 |
| | taagaaggagatatacatatgAACTGGGTGAACGTTATCAGCG | |
| | Reverse primer 1 for Analog 17: | 72 |
| | atgatggtgagtttctggacgaacGCTGGTGTTAATAAACATTTGAAC G | |
| | Reverse primer 2 for Analog 17: | 73 |
| | gtggtggtggtggtgctcgagTTAATGATGGTGAGTTTCTGGACGAA | |
| Analo g 18 | IL-15-KKK | |
| | IL-15-aaaaaaaag | |
| | Forward primer for Analog 18: | 74 |
| | taagaaggagatatacatatgAACTGGGTGAACGTTATCAGCG | |
| | Reverse primer 1 for Analog 18: | 75 |
| | cttttttttGCTGGTGTTAATAAACATTTGAACG | |
| | Reverse primer 2 for Analog 18: | 76 |
| | gtggtggtggtggtgctcgagTTACTTTTTTTTGCTGGTGTTAATAAA CA | |
| Analo g 19 | IL-15-RHHHH | |
| | IL-15-cgtcaccatcatcat | |
| | Forward primer for Analog 19: | 77 |
| | taagaaggagatatacatatgAACTGGGTGAACGTTATCAGCG | |
| | Reverse primer 1 for Analog 19: | 78 |
| | atgatgatggtgacgGCTGGTGTTAATAAACATTTGAACG | |
| | Reverse primer 2 for Analog 19: | 79 |
| | gtggtggtggtggtgctcgagTTAATGATGATGGTGACGGCTGG | |
| Analo g 20 | IL-15-KRETHHHH | |
| | IL-15-aagcgtgaaactcaccatcatcat | |
| | Forward primer for Analog 20: | 80 |
| | taagaaggagatatacatatgAACTGGGTGAACGTTATCAGCG | |
| | Reverse primer 1 for Analog 20: | 81 |
| | atgatgatggtgagtttcacgcttGCTGGTGTTAATAAACATTTGAACG | |
| | Reverse primer 2 for Analog 20: | 82 |
| | gtggtggtggtggtgctcgagTTAATGATGATGGTGAGTTTCACGCT | |
| Analo g 21 | IL-15-GS-LPETG-GSGGSHHHHHH | |
| | IL-15-ggttctctgccggaaaccggtggttctggtggttctcaccaccaccaccaccac | |
| | Forward primer for Analog 21: | 83 |
| | taagaaggagatatacatatgAACTGGGTGAACGTTATCAGCG | |
| | Reverse primer 1 for Analog 21: | 84 |
| | accaccagaaccaccggtttccggcagagaaccGCTGGTGTTAATAAAC ATT | |
| | Reverse primer 2 for Analog 21: | 85 |
| | gtggtggtggtggtgctcgagTTAGTGGTGGTGGTGGTGAGAA CCACCAGAACC | |
| Analo g 22 | IL-15-HLETGKKK | |
| | IL-15-caccttgaaactggtaaaaagaag | |
| | Forward primer for Analog 22: | 86 |
| | taagaaggagatatacatatgAACTGGGTGAACGTTATCAGCG | |
| | Reverse primer 1 for Analog 22: | 87 |
| | cttctttttaccagtttcaaggtgGCTGGTGTTAATAAACATTTGAACG | |
| | Reverse primer 2 for Analog 22: | 88 |
| | gtggtggtggtggtgctcgagTTACTTCTTTTTACCAGTTTCAAGGTG G | |
| Analo g 23 | IL-15-HVESGRRR | |
| | IL-15-catgttgaatctggtcgtcgccgt | |
| | Forward primer for Analog 23: | 89 |
| | taagaaggagatatacatatgAACTGGGTGAACGTTATCAGCG | |
| | Reverse primer 1 for Analog 23: | 90 |
| | acggcgacgaccagattcaacatgGCTGGTGTTAATAAACATTTGAA CG | |
| | Reverse primer 2 for Analog 23: | 91 |
| | gtggtggtggtggtgctcgagTTAACGGCGACGACCAGATTCA | |
| Analo g 24 | IL-15-RRHTGKKK | |
| | IL-15-cgtcgtcatactggtaaaaagaag | |
| | Forward primer for Analog 24: | 92 |
| | taagaaggagatatacatatgAACTGGGTGAACGTTATCAGCG | |
| | Reverse primer 1 for Analog 24: | 93 |
| | cttctttttaccagtatgacgacgGCTGGTGTTAATAAACATTTGAACG | |
| | Reverse primer 2 for Analog 24: | 94 |
| | gtggtggtggtggtgctcgagTTACTTCTTTTTACCAGTATGACGAC GG | |
| Analo g 25 | IL-15-HVKTGHHH | |
| | IL-15-catgttaagactggtcaccatcat | |
| | Forward primer for Analog 25: | 95 |
| | taagaaggagatatacatatgAACTGGGTGAACGTTATCAGCG | |
| | Reverse primer 1 for Analog 25: | 96 |
| | atgatggtgaccagtcttaacatgGCTGGTGTTAATAAACATTTGAAC G | |
| | Reverse primer 2 for Analog 25: | 97 |
| | gtggtggtggtggtgctcgagTTAATGATGGTGACCAGTCTTAACAT GG | |
| Analo g 26 | IL-15-HVKSGRHH | |
| | IL-15-catgttaagtctggtcgtcatcat | |
| | Forward primer for Analog 26: | 98 |
| | taagaaggagatatacatatgAACTGGGTGAACGTTATCAGCG | |
| | Reverse primer 1 for Analog 26: | 99 |
| | atgatgacgaccagacttaacatgGCTGGTGTTAATAAACATTTGAAC G | |
| | Reverse primer 2 for Analog 26: | 100 |
| | gtggtggtggtggtgctcgagTTAATGATGACGACCAGACTTAACAT GG | |
| Analo g 27 | IL-15-HVKSSHRH | |
| | IL-15-catgttaagtctagccatcgtcac | |
| | Forward primer for Analog 27: | 101 |
| | taagaaggagatatacatatgAACTGGGTGAACGTTATCAGCG | |
| | Reverse primer 1 for Analog 27: | 102 |
| | gtgacgatggctagacttaacatgGCTGGTGTTAATAAACATTTGAAC G | |
| | Reverse primer 2 for Analog 27: | 103 |
| | gtggtggtggtggtgctcgagTTAGTGACGATGGCTAGACTTAACAT GG | |
| Analo g 28 | IL-15-(GS)₅-LVKSGHHH | |
| | IL-15-ggtagcggtagcggtagcggtagcggtagcctggtaaagtctggtcaccatcat | |
| | Forward primer for Analog 28: | 104 |
| | taagaaggagatatacatatgAACTGGGTGAACGTTATCAGCG | |
| | Reverse primer 1 for Analog 28: | 105 |
| | gctaccgctaccgctaccgctaccgctaccGCTGGTGTTAATAAACATTT GAACG | |
| | Reverse primer 2 for Analog 28: | 106 |
| | atgatggtgaccagactttaccagGCTACCGCTACCGCTACCG | |
| | Analog 28 Reverse primer for 3: | 107 |
| | gtggtggtggtggtgctcgagTTAATGATGGTGACCAGACTTTACCA G | |
| Analo g 29 | IL-15-RPKSGHHK | |
| | IL-15-cgtccaaagagcggtcaccataag | |
| | Forward primer for Analog 29: | 108 |
| | taagaaggagatatacatatgAACTGGGTGAACGTTATCAGCG | |
| | Reverse primer 1 for Analog 29: | 109 |
| | cttatggtgaccgctctttggacgGCTGGTGTTAATAAACATTTGAACG | |
| | Reverse primer 2 for Analog 29: | 110 |
| | gtggtggtggtggtgctcgagTTACTTATGGTGACCGCTCTTTGGA | |
| Analo g 30 | IL-15-KKC | |
| | IL-15-aaaaagtgt | |
| | Forward primer for Analog 30: | 111 |
| | taagaaggagatatacatatgAACTGGGTGAACGTTATCAGCG | |
| | Reverse primer for Analog 30: | 112 |
| | gtggtggtggtggtgctcgagTTAacactttttGCTGGTGTTAATAAACAT TTGAACG | |
| Analo g 31 | IL-15-LHKAGKHH | |
| | IL-15-cttcacaaggctggtaaacaccat | |
| | Forward primer for Analog 31: | 113 |
| | taagaaggagatatacatatgAACTGGGTGAACGTTATCAGCG | |
| | Reverse primer 1 for Analog 31: | 114 |
| | atggtgtttaccagccttgtgaagGCTGGTGTTAATAAACATTTGAACG | |
| | Reverse primer 2 for Analog 31: | 115 |
| | gtggtggtggtggtgctcgagTTAATGGTGTTTACCAGCCTTGTGAA | |
| Analo g 32 | IL-15-K | |
| | IL-15-aaa | |
| | Forward primer for Analog 32: | 116 |
| | taagaaggagatatacatatgAACTGGGTGAACGTTATCAGCG | |
| | Reverse primer for Analog 32: | 117 |
| | gtggtggtggtggtgctcgagTTAtttGCTGGTGTTAATAAACATTTGA ACG | |
| Analo g 33 | IL-15-KK | |
| | IL-15-aaaaag | |
| | Forward primer for Analog 33: | 118 |
| | taagaaggagatatacatatgAACTGGGTGAACGTTATCAGCG | |
| | Reverse primer for Analog 33: | 119 |
| | gtggtggtggtggtgctcgagTTActttttGCTGGTGTTAATAAACATTTG AACG | |

The analogs 34 to 43 were formed by introducing mutations into the wild-type IL-15 to form IL-15 mutants, and then adding the sequence GSLPETGGGSGGSHHHHHH to the C-terminals based on the IL-15 mutants. The analogs 34- to 43- were the mutants of IL-15 formed just after the mutations were introduced, without further adding the sequence GSLPETGGGSGGSHHHHHH to the C-terminals. The nucleotide sequences of the mutated analogs 34 to 43 and analogs 38- to 43- without adding the sequence GSLPETGGGSGGSHHHHHH to the C-terminals were synthesized by the Genewiz, Inc., and cloned into the pET41a vectors.

| Name | Sequence of Protein | SE Q ID NO. | Identit y with IL-15 | SEQ ID NO. (Amino Acid Sequence) |
|---|---|---|---|---|
| IL-15 | | 1 | | 2 |
| Analo g 34 | MNWVNVISDLKKIEDLIQSMHIDATLYTES DVHPSCKVTAMKCFLLELQVISLESGDAS IHDTVENLIILANNSLSSNANVTESGCKEC EELEEKNIKEFLQSFVHIVQMFINTSGSLP ETGGSGGSHHHHHH | 3 | 99% | 13 |
| Analo g 35 | MANWVNVISDLKKIEDLIQSMHIDATLYTE SDVHPSCKVTAMKCFLLELQVISLESGDA SIHDTVENLIILANNSLSSNANVTESGCKE CEELEEKNIKEFLQSFVHIVQMFINTSGSL PETGGSGGSHHHHHH | 4 | 97% | 14 |
| Analo g 36 | MNWVNVISDLKKIEDLIQSMHIRGDLYTE SDVHPSCKVTAMKCFLLELQVISLESGDA SIHDTVENLIILANNSLSSNANVTESGCKE CEELEEKNIKEFLQSFVHIVQMFINTSGSL PETGGSGGSHHHHHH | 5 | 96% | 15 |
| Analo g 37 | MSNWVNVISDLKKIEDLIQSVHIRGDLYTE SDVHPSCKVTAMKCFLLELQVISLESGDG SIHDTVENLIILAQQSLSSNANVTESGCKE CEELEEKNIKEFLQSFVHIVQMFINTSGSL PETGGSGGSHHHHHH | 6 | 93% | 16 |
| Analo g 38 | MSNWVNVISDLKKIEDLIQSVHIRGDLYTE SDVHPSCKVTAMKCFLLELQVISLESGDG | 7 | 90% | 17 |
| | SIHDTVENLIILAQQSLSSNANVTESGCKE CEELSEKNIKEFLQSFVHIVQVFINTSGSL PETGGSGGSHHHHHH | | | |
| Analo g 39 | MSNWVNVISDLRKIRGDIQSVHIDATLYT ESDVHPSCRVTAMKCFLLELQVISLESGD ASIHDTVENLIILANQSLSSNANVTESGCK ECEELEEKNIKEFLQSFVHIVQLFIQTSGS LPETGGSGGSHHHHHH | 8 | 90% | 18 |
| Analo g 40 | MSNWVNVISDLRKIRGDLNAVHVDATLYT ESDVHPSCRVTAMKCFLLELQVISLESGD ASIHDTVENLIILANQSLSSNANVTESGCK ECEELEEKNIKEFLQSFVHIVQLFIQTSGS LPETGGSGGSHHHHHH | 9 | 85% | 19 |
| Analo g41 | MSNWVNVISDLRKIRGDIQSVHIDATLYT ESDVHPSCRVTAMKCFLLELQVISLESGD ASIHDTVENLIILANQSLAAQAQLTESGCK ECEELEEKNIKEFLQSFVHIVQLFIQTSGS LPETGGSGGSHHHHHH | 10 | 85% | 20 |
| Analo g 42 | MSNWVNVISDLRKIRGDLNAVHVDATLYT ESDVHPSCRVTAMKCFLLELQVISLESGD ASIHDTVENLIILANQSLAAQAQLTESGCK ECEELEEKNIKEFLQSFVHIVQLFIQTSGS LPETGGSGGSHHHHHH | 11 | 80% | 21 |
| Analo g 43 | MSNWVNVISDLRKIRGDIQSVHIDATLYT ESDVHPSCRVTAMKCFLLELQLISLDSGD ASIHETVEQLILLANQSLAAQAQLTESGC KECEELEEKNIKEFLQSFVHIVQLFIQTSG SLPETGGSGGSHHHHHH | 12 | 80% | 22 |

### (2) PCR amplification

The PCR amplification was classified into three cases as follows.

In the first case, only one reverse primer was required, and a total of 1 run of PCR was performed.
Sample loading system: 50 µL (1 tube)
Primers: forward primer and reverse primer
Template: IL-15
Annealing temperature: 61 °C
Extension time: 30 s

In the second case, two reverse primers were required, and a total of 2 runs of PCR were performed.
1^{st} run of PCR
Sample loading system: 20 µL (1 tube)
Primers: forward primer and reverse primer 1
Template: IL-15
Annealing temperature: 61 °C
Extension time: 30 s
2^{nd} run of PCR
Sample loading system: 50 µL (1 tube)
Primers: forward primer and reverse primer 2
Template: PCR product of 1^{st} run
Annealing temperature: 61 °C
Extension time: 30 s

In the third case, three reverse primers were required, and a total of 3 runs of PCR were performed.
1^{st} run of PCR
Sample loading system: 20 µL (1 tube)
Primers: forward primer and reverse primer 1
Template: IL-15
Annealing temperature: 61 °C
Extension time: 30 s
2^{nd} run of PCR
Sample loading system: 20 µL (1 tube)
Primers: forward primer and reverse primer 2
Template: PCR product of 1^{st} run
Annealing temperature: 61 °C
Extension time: 30 s
3^{rd} run of PCR
Sample loading system: 50 µL (1 tube)
Primers: forward primer and reverse primer 3
Template: PCR product of 2^{nd} run
Annealing temperature: 61 °C
Extension time: 30 s
Sample loading system for PCR: 20 µL

| | |
|---|---|
| 5×*TransStart*^{®} FastPfu Fly buffer | 4 µL |
| Forward primer (5 µM) | 1.2 µL |
| Reverse primer (5 µM) | 1.2 µL |
| dNTPs (2.5 mM) | 1.6 µL |
| Template | 0.5 µL |
| Pfu DNA polymerase | 0.4 µL |
| ddH₂O | making up to 20 µL |
| Sample loading system for PCR: | 50 µL |
| 5×*TransStart*^{®} FastPfu Fly buffer | 10 µL |
| Forward primer (5 µM) | 3 µL |
| Reverse primer (5 µM) | 3 µL |
| dNTPs (2.5 mM) | 4 µL |
| Template | 0.5 µL |
| Pfu DNA polymerase | 1 µL |
| ddH₂O | making up to 50 µL |

Amplification procedure for PCR:

| | | |
|---|---|---|
| 98 °C | 5 min | 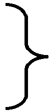 |
| 98 °C | 20 s | |
| 61 °C | 20 s | |
| 72 °C | 30 s | |
| 72 °C | 10 min | |

### (3) Enzyme digestion of vector

Reaction system: 30 µL, incubating at 37 °C overnight after mixing

| | |
|---|---|
| pET41a vector | 5 µg |
| *Nde*I | 1 µL |
| *Xho*I | 1 µL |
| 10×H buffer | 3 µL |
| ddH₂O | making up to 30 µL |

### (4) PCR products and digested vectors in the gels were recovered.

### (5) Loading of recombinants

Reaction system: 20 µL

| | |
|---|---|
| Digested pET41a vector | 13.5 µL |
| PCR product | 0.5 µL |
| 5× CE II buffer | 4 µL |
| Exnase II | 2 µL |

The molar ratio of vector to fragment was 2:1. After reacting at 37°C for 30 min, it was immediately cooled on ice and transformed into DH5α.

### (6) Sterility test

0.5 mL of Amp-resistant LB media was added to a 1.5 mL centrifuge tube and well-grown single colonies were inoculated thereto. A total of 5 tubes were inoculated and they were incubated in a shaker under shaking at 37°C. After incubation for 3 h, 1 µL of the culture was taken as a template for PCR of the bacterial suspension.
Reaction system for PCR: 10 µL

| | |
|---|---|
| 10×Taq DNA polymerase buffer | 1 µL |
| T7-P (5µM) | 1 µL |
| T7-T (5µM) | 1 µL |
| dNTPs (2.5mM) | 0.8 µL |
| Template | 1 µL |
| Taq DNA polymerase | 0.1 µL |
| ddH₂O | 5.1 µL |

Amplification procedure for PCR:

| | | |
|---|---|---|
| 94 °C | 5 min | 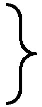 |
| 94 °C | 30 s | |
| 55 °C | 30 s | |
| 72 °C | 1 min | |
| 72 °C | 5 min | |

After PCR was completed, it was detected by agarose gel electrophoresis. Three positive clones were selected for sequencing (Sangon Biotech).

### (7) Preservation of positive bacterial suspension and extraction of plasmids

The positive clones with correct sequencing were inoculated into 5 mL of Amp-resistant LB liquid media with an inoculation volume of 10 µL. 500 µL of bacterial suspension was added into a 1.5 mL centrifuge tube. 500 µL of 40% glycerol was added for storage. It was labeled with the name, host bacteria and date, capped and stored in a refrigerator at -80 °C.

The remaining bacterial suspension was collected by centrifugation for plasmid extraction.

### 1.2 Protein expression of wild-type IL-15 and IL-15 analogs

### (1) Transformation of BL21 (DE3)

a) 2 µL of plasmid was added to 100 µL of BL21 (DE3) competent cells, and it was mixed immediately and placed on ice for 30 min.
b) It was heat-shocked at 42 °C for 90 s, followed by a rapid ice bath for 2 min.
c) 500 µL of LB media was added, then it was incubated at 37 °C under shaking (≤200 rpm) for 60 minutes.
d) It was centrifuged at 6000 rpm for 1 min. Most of the supernatant was discarded, and about 100 to 150 µL of the supernatant was retained. After the pellet was resuspended, they were spread on LB plates containing Amp and cultured at 37 °C overnight.

### (2) Small-scale expression

a) Bacteria preservation: One single clone was picked into 1 mL of Amp-resistant LB media. It was incubated at 37 °C under shaking at 220 rpm for about 5 h. 1 mL of 40% glycerol was added. It was divided into 2 tubes and cryopreserved at -80 °C.
b) 2.5 mL of LB liquid media containing Amp was added to the tube in the previous step. The culture was incubated at 37 °C under shaking at 220 rpm overnight.
c) The bacterial suspension incubated overnight was inoculated into 20 mL of LB media containing Amp at a ratio of 1:50. It was incubated at 37 °C under shaking at 220 rpm to reach OD600=0.6 (about 3h). IPTG was added at a final concentration of 0.5 mM. It was incubated at 37 °C under shaking at 220 rpm for 3 h.

### (3) Expression level determination by SDS-PAGE

a) The cultural suspension was determined for OD600. 10OD bacterial suspension was taken, and centrifuged at 10000 rpm for 2min. The supernatant was removed.
b) The pellet was resuspended with 1 mL of lysis buffer (10 mM Tris-HCl, pH 8.0) placed on ice and lysed by ultrasonication. Ultrasonic conditions: 130 W, 4min, on 3s, off 3s.
c) After ultrasonication, 80 µL was taken and labeled as "T (total)". The remaining liquid was centrifuged at 12000 rpm for 10 min to obtain "S (supernatant)" and "P (pellet)". 20 µL of 5×Reducing Loading Buffer was added to 80 µL of T, P and S, respectively. Then they were heated at 95 °C for 5 min and 12.5 µL (0.1 OD) of each sample was taken for SDS-PAGE electrophoresis.

The electrophoretogram of wild-type IL-15 was shown in Fig. 1, wherein the expression of wild-type IL-15 was virtually undetectable in "T (total)", "S (supernatant)" or "P (pellet)". The electrophoretograms of IL-15 analogs 1 to 33 were shown in Figs. 2a-2e, wherein the expression levels of the IL-15 analogs were significantly higher than that of wild-type IL-15. Further, the expression levels of the IL-15 analogs (analogs 34 to 43) with the sequence GSLPETGGGSGGSHHHHHH at the C-terminal were also significantly higher than those of the IL-15 analogs (analogs 38- to 43-) without the sequence GSLPETGGGSGGSHHHHHH at the C-terminal, as shown in Figs. 2f and 2g.

### (4) Expression level determination by HPLC

a) After expression, 10OD cells were collected and resuspended with 1 mL of 10 mM Tris-HCl buffer at pH 8.0.
b) Sonication was performed under the same conditions as those for running gel electrophoresis as described in the above step (3).
c) After sonication, it was centrifuged at 12000 rpm for 10 min. The supernatant was discarded.
d) The pellet was added with 1 mL of freshly prepared 8 M Urea/10 mM Tris-HCl (pH 8.0, 10 mM DTT) and dissolved by shaking at room temperature for about 1 h.
e) It was filtered with a 0.2 µm filter and loaded for HPLC analysis. The analysis was performed using a C4 analytical column with 0.1% TFA in deionized water as mobile phase A and 0.1% TFA in acetonitrile as mobile phase B, followed by a 15-minute gradient from 20% B to 60% B.

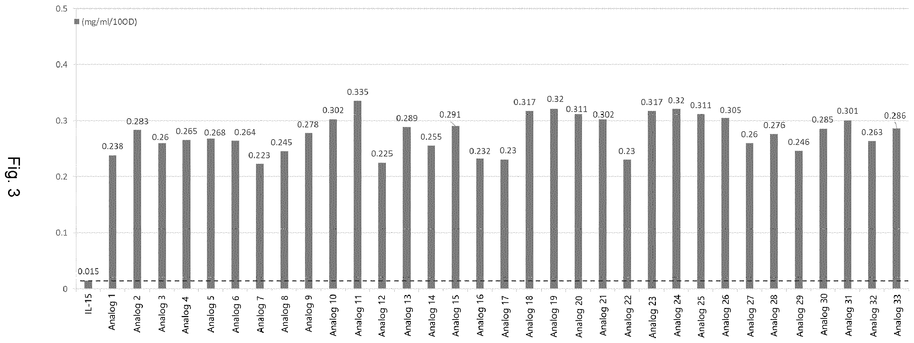

As shown by the HPLC quantitative results in Fig. 3, the expression levels of the IL-15 analogs 1 to 33 were about 20-fold higher than that of the wild-type IL-15.

### Example 2: In vitro cell activity assay of IL-15 analogs

### 2.1 Preparation of IL-15 analogs

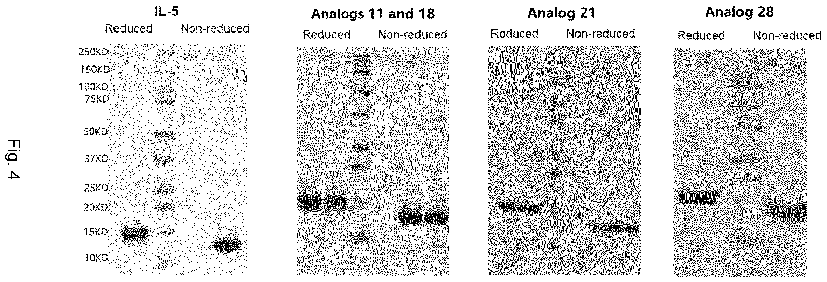

The IL-15 analogs 11, 18, 21 and 28 expressed by the inclusion bodies were dissolved in 8 M urea solution, and purified by ion exchange and reversed-phase chromatography (for details, see: Yunier Rodríguez-Álvarez et al, Preparative Biochemistry and Biotechnology, 47: 9, 889-900), to obtain relatively pure proteins. The SDS-PAGE electrophoretograms were shown in Fig. 4.

### 2.2 CTLL-2 cell proliferation assay

The CTLL-2 cell proliferation assay is commonly used to detect the activity of immune cells stimulated by interleukin at the cellular level. Therefore, the biological activity of IL-15 analogs was determined herein by the proliferative effect of the wild-type IL-15 and IL-15 analogs on CTLL-2 cells.
1) Preparation of CTLL-2 cells: the cells were resuspended in media containing FBS and Rat-T-Stim.
2) Loading: the cells were seeded in a 96-well culture plate at 0.1 mL per well. At the same time, the proteins samples of IL-15 analogs 11, 18, 21 and 28 to be tested (i.e., the proteins prepared in step 2.1) were diluted by multiples, respectively. 0.1 mL was added to each well, and 3 replicate wells were set for each dilution concentration. The control well for culture media was set (100 µL cells + 100 µL culture media). The plate was incubated at 37 °C with 5% CO₂ for 72 hours.
3) MTS addition: 20 µL of CellTiter96^{®} AQueous One Solution Reagent was added to each well, and the plate was incubated at 37 °C with 5% CO₂ for 2 to 4 hours.
4) Detection: the absorbance value (A) was measured at a wavelength of 490 nm with a microplate reader and the EC50 value was calculated.

The results were shown in Table 3. There was no significant difference in the cellular activity of the IL-15 analogs and wild-type IL-15.

**Table 3: CTLL-2 cell viability assay**

| | EC50 (ng/mL) |
|---|---|
| IL-15 | 0.05445 |
| Analog 11 | 0.05252 |
| Analog 18 | 0.05609 |
| Analog 21 | 0.05235 |
| Analog 28 | 0.05085 |

### Example 3: Preparation of conjugates of IL-15 analogs (Enzymatic Reaction Method)

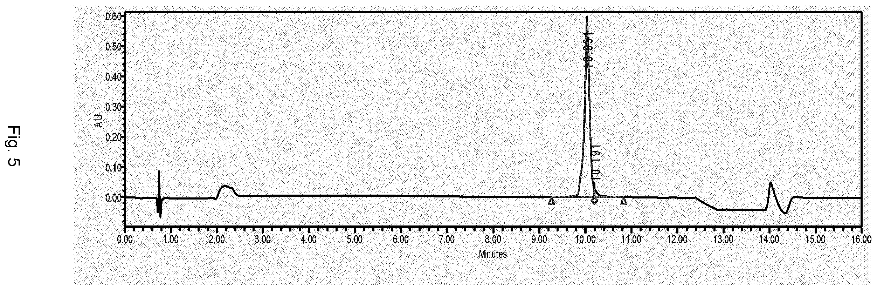

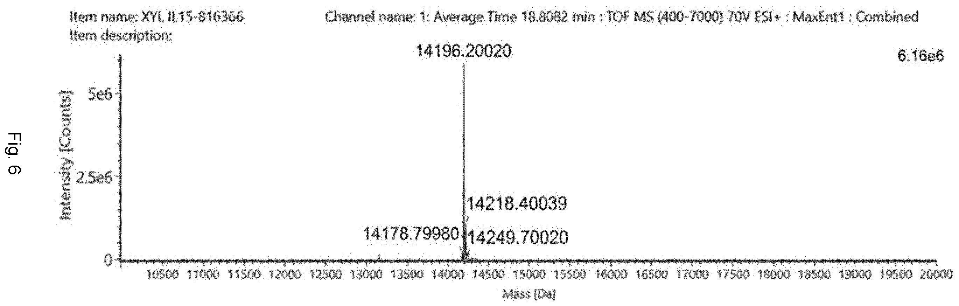

The purified IL-15 Analog 21 (IL-15-GS-LPETG-GSGGSHHHHHH) was used in this example. The fatty acid chain (816366) with GGG at the N-terminal was linked to IL-15-GS-LPETG- GSGGSHHHHHH by a ligation reaction catalyzed by the transpeptidase Sortase A. The reaction was carried out in a Sortase A: IL-15: fatty acid chain ratio of 1: 6: 30, wherein the reaction buffer was 50 mM Tris-HCl (1 mM CaCl, 150 mM NaCl, pH 8.0). After reacting at room temperature for 3 hours, purification was carried out. Reversed-phase chromatography C8 (Sepax Technologies, Inc.) was used for purification to separate the unconjugated IL-15 Analog 21 and the unreacted fatty acid chains from the conjugated products. The purity of the final product was determined by UPLC (Fig. 5) and LC-MS (Fig. 6). The results showed that IL-15 Analog 21 had been coupled to a fatty acid chain to form IL-15 Analog 21-816366.

### Example 4: Preparation of conjugates of IL-15 analog (coupling the N-terminal amino group of the IL-15 analog to a fatty acid chain)

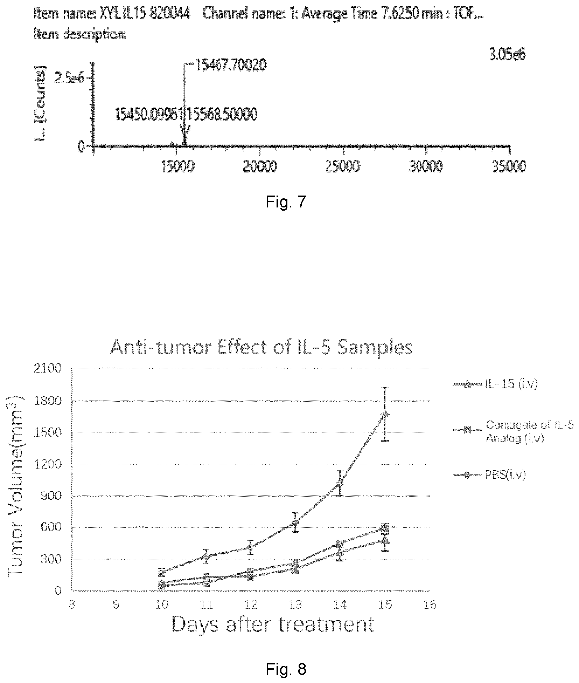

The purified IL-15 Analog 21 was coupled to a fatty acid chain with a succinimidyl ester (820044) through the amino group at N-terminal thereof at neutral pH. The reaction was carried out in an IL-15: fatty acid chain ratio of 1: 1, wherein the reaction buffer was PBS at pH 7.2. After reacting at room temperature for 1 hour, purification was carried out. Reversed-phase chromatography C8 (Sepax Technologies, Inc.) was used for purification to separate the unconjugated IL-15 and the unreacted fatty acid chains from the conjugated products. The final product was identified by LC-MS. As shown in Fig. 7, IL-15 has been coupled to a fatty acid chain to form the IL-15 Analog 21-820044.

### Example 5: Binding assay of the IL-15 Analog 21 and conjugates of IL-15 Analog 21 to the IL15Rα Receptor

The conjugates of IL-15 analogs used in this example were prepared in Example 3.

In this example, biolayer interferomeory (BLI) was used to determine the affinity between the target protein and the receptor. For procedures, see Patricia Estep et al., High throughput solution Based measurement of antibody-antigen affinity and epitope binning, MAbs 2013, 5(2): 270-278. The receptor protein IL15Rα-His used in the experiment was produced by Leto Laboratories Co. Ltd. The formulation of buffer was: 10 mM HEPES, 150 mM NaCl, 3 mM EDTA, and 0.05% Tween 20. The receptor IL15Rα-His was pre-immobilized on a HISIK sensor (Pall Fortebio, Catalog# 18-5120), followed by an established process comprising the steps of setting baseline, loading, baseline, association and dissociation. Data acquisition and analysis were carried out using the software Data acquisition 11.0 and Data analysis 11.0 installed with Octet RED96, respectively.

The results for affinity assay of the IL-15 analog and the conjugated products of IL-15 analogs and fatty acid chains to the IL15Rα receptors are shown in Table 4. As compared to the IL-15 analog before conjugation, the affinity of the conjugated products of IL-15 analog and fatty acid chains to the IL15Rα receptor did not change significantly.

**Table 4: Results for affinity assay**

| Name of Protein | Affinity to IL15Rα (M) |
|---|---|
| IL-15Analog 21 | 1.18E-09 |
| IL-15Analog 21-816366 | 6.6E-09 |
| IL-15Analog 21-823479 | 4E-09 |
| IL-15Analog 21-823480 | 8E-10 |

### Example 6: Half-life of IL-15 Analog 21 and conjugates of IL-15 Analog 21 in mice

The conjugates of IL-15 analogs used in this example were prepared in Example 3.

Eight C57BL/6 mice were divided into 2 groups with 4 mice in each group. Blood was collected from two mice at each time point, and blood was collected cyclically. For one group, the mice were injected with IL-15 at 0.5 mg/kg via tail vein. Blood samples were collected immediately, and then at 10 min, 30 min, 1.5 h and 4 h after administration. For the other group, the mice were injected with long-acting IL-15 at 0.5 mg/kg via tail vein. Blood samples were collected immediately, and then at 1 h, 2 h and 4 h after administration. At each time point, 50 to 100 µL of blood was collected from eye orbit. Then serum was collected for ELISA assay of IL-15.

The calculation formula of half-life is: t_{1/2} = 0.693/k, k = (Inc₀-Inc)/t. Upon calculation, the half-life of IL-15 was about 5 min, and the half-life of long-acting IL-15 could reach 1.5 h.

### Example 7: In vivo tumor inhibition assay for IL-15 Analog 21 and conjugates of IL-15 analog in mice

The conjugates of IL-15 analogs used in this example were prepared in Example 3.

Fifteen C57BL/6 mice aged 6 to 8 weeks were randomly divided into 3 groups, namely the reagent control group (PBS), the IL-15 group and the long-acting IL-15 group, with 5 mice in each group. On Day 1, the mice were subcutaneously inoculated with B16-F10 cells on the back of the neck, with 2×10⁵/100 µL/mouse. On Days 4 to 8, the PBS group and the IL-15 group were administered intravenously (i. v.) for 5 consecutive days, respectively, at a dose of 20 µg/100 µL/mouse (IL-15 group) or 100 µL/mouse (PBS group). The IL-15 analog conjugate group was administered twice in the same way on Day 4 and Day 7, with the same dosage of 20 µg/100 µL/mouse each time. Tumor sizes were observed from Day 10 and continued for 6 days.

The results are shown in Fig. 8. As compared to the control group, both IL-15 and long-acting IL-15 exhibited significant tumor-inhibiting effects.

## Claims

1. An IL-15 analog, wherein the amino acid sequence of the IL-15 analog comprises the amino acid sequence of IL-15, and one or more amino acids added to the C-terminal of the amino acid sequence of IL-15,
wherein the one or more amino acids added to the C-terminal of the amino acid sequence of IL-15 are selected from the group of:
GSGSGS-HHHHHH, GS-HHHHHH, PLASTKKR, LPKSAKKK, KKKKKKK, GAPQGAPQ-LVESAHHH, GS-LVSSAHHK, GS-LIEHHRRK, GS-IVEHRKKK, GS-VPKTGRRR, GS-LVASGKK, GS-HRKSGHHH, GS-LPKTGRHK, KKKTGRRH, LPRSGRHK, LVETHHHH, VRPETHHH, KKK, RHHHH, KRETHHHHH, GS-LPETG-GSGGSHHHHHH HLETGKKK, HVESGRRR, RRHTGKKK, HVKTGHHH, HVKSGRHH, HVKSSHRH, GSGSGSGSGS-LVKSGHHH, RPKSGHHK, KKC, LHKAGKHH, K, and KK, and wherein the amino acid sequence of IL-15 is shown in SEQ ID NO. 1.

2. A nucleotide sequence encoding the IL-15 analog of claim 1.

3. A method for preparing an IL-15 analog, wherein the IL-15 analog of claim 1 is expressed in a prokaryotic system.

4. The method for preparing the IL-15 analog of claim 3, wherein a nucleotide sequence encoding the IL-15 analog is linked to a prokaryotic expression vector, and transferred into a prokaryotic expression host bacterium, which is induced to express the IL-15 analog.

5. A recombinant expression vector, comprising a nucleotide sequence encoding the IL-15 analog of claim 1.

6. A host bacterium transformed with a nucleotide sequence encoding the IL-15 analog of claim 1.

7. A conjugate of an IL-15 analog, wherein the amino acid sequence of the IL-15 analog comprises the amino acid sequence of IL-15, and one or more amino acids added to the C-terminal of the amino acid sequence of IL-15; and wherein the conjugate of the IL-15 analog comprises the IL-15 analog of claim 1 and a fatty acid chain.

8. The conjugate of the IL-15 analog of claim 7 wherein the fatty acid chain is-(CH₂)ₘ-COOH, wherein m is 12 to 19.

9. The conjugate of the IL-15 analog of claim 7 wherein the IL-15 analog and the fatty acid chain are linked by *in vitro* coupling.

10. The conjugate of the IL-15 analog of claim 9, wherein the *in vitro* coupling comprises a method selected from the group of:
1) coupling through enzymatic reaction, wherein the fatty acid chain in the enzymatic reaction has GGG at the N-terminal;
2) coupling with free cysteine residues introduced into the IL-15 analog; and
3) coupling with the amino group at the N-terminal of the IL-15 analog.

11. The conjugate of the IL-15 analog of claim 10, wherein
when coupling through enzymatic reaction, the fatty acid chain has 3 glycine residues at the terminal;
when coupling with free cysteine residues introduced into the IL-15 analog, the fatty acid chain has a maleimide ester or a halogenated reactive group; and
when coupling with the amino group at the N-terminal of the IL-15 analog, the fatty acid chain has an aldehyde group or a succinimidyl ester functional group.

12. The conjugate of the IL-15 analog of claim 7, wherein the IL-15 analog is obtained by adding a sequence comprising -GS-LPETG to the terminal of the amino acid sequence of IL-15.

13. The conjugate of the IL-15 analog of claim 7, wherein the fatty acid chain is selected from the group consisting of:
GGG-PEG2-Lys-(CH₂)₁₆-COOH,
NHS-PEG2-PEG2-γ-Glu-(CH₂)₁₇-COOH,
GGG-PEG4-PEG4-PEG4-Lys-(CH₂)₁₇-COOH,
GGG-PEG4-γ-Glu-γ-Glu-Lys-(CH₂)₁₇-COOH,
HOOC-(CH₂)₁₆-γ-Glu-γ-Glu-Lys-GGG,
HOOC-(CH₂)₁₆-CONH-γ-Glu-γ-Glu-PEG2-Lys-Br, GGG-γ-Glu-C2DA-2OEG-γ-Glu-(CH₂)₁₇-COOH, GGG-γ-Glu-C2DA-20EG-γ-Glu-(CH₂)₁₉-COOH, GGG-OEG-C2DA-20EG-γ-Glu-(CH₂)₁₉-COOH, GGG-OEG-C2DA-2OEG-γ-Glu-Trx-(CH₂)₁₉-COOH,
CHO-PEG2-PEG2-γ-Glu-(CH₂)₁₇-COOH, and
Mal-C2DA-2OEG-γ-Glu-Tn-(CH₂)₁₉-COOH.

## Patentansprüche

1. Ein IL-15-Analogon, wobei die Aminosäuresequenz des IL-15-Analogons die Aminosäuresequenz von IL-15 und eine oder mehrere Aminosäuren, die an den C-Terminus der Aminosäuresequenz von IL-15 angefügt sind, umfasst,
wobei die eine oder die mehreren Aminosäuren, die an den C-Terminus der Aminosäuresequenz von IL-15 angefügt sind, ausgewählt sind aus der Gruppe bestehend aus:
GSGSGS-HHHHHH, GS-HHHHHH, PLASTKKR, LPKSAKKK, KKKKKKK, GAPQGAPQ-LVESAHHH, GS-LVSSAHHK, GS-LIEHHRRK, GS-IVEHRKKK, GS-VPKTGRRR, GS-LVASGKK, GS-HRKSGHHH, GS-LPKTGRHK, KKKTGRRH, LPRSGRHK, LVETHHHH, VRPETHHH, KKK, RHHHH, KRETHHHHH, GS-LPETG-GSGGSHHHHHH, HLETGKKK, HVESGRRR, RRHTGKKK, HVKTGHHH, HVKSGRHH, HVKSSHRH, GSGSGSGSGS-LVKSGHHH, RPKSGHHK, KKC, LHKAGKHH, K, und KK, und wobei die Aminosäuresequenz von IL-15 in SEQ-ID Nr. 1 gezeigt ist.

2. Nukleotidsequenz, die für das IL-15-Analogon nach Anspruch 1 kodiert.

3. Verfahren zur Herstellung eines IL-15-Analogons, wobei das IL-15-Analogon nach Anspruch 1 in einem prokaryotischen System exprimiert wird.

4. Verfahren zur Herstellung des IL-15-Analogons nach Anspruch 3, wobei eine Nukleotidsequenz, die für das IL-15-Analogon kodiert, mit einem prokaryotischen Expressionsvektor verbunden und in ein prokaryotisches Expressionswirtsbakterium übertragen wird, das zur Expression des IL-15-Analogons induziert wird.

5. Rekombinanter Expressionsvektor, umfassend eine Nukleotidsequenz, die für das IL-15-Analogon nach Anspruch 1 kodiert.

6. Wirtsbakterium, das mit einer Nukleotidsequenz transformiert ist, die für das IL-15-Analogon nach Anspruch 1 kodiert.

7. Konjugat eines IL-15-Analogons, wobei die Aminosäuresequenz des IL-15-Analogons die Aminosäuresequenz von IL-15 und eine oder mehrere Aminosäuren, die dem C-Terminus der Aminosäuresequenz von IL-15 hinzugefügt sind, umfasst; und wobei das Konjugat des IL-15-Analogons das IL-15-Analogon nach Anspruch 1 und eine Fettsäurekette umfasst.

8. Konjugat des IL-15-Analogons nach Anspruch 7, wobei die Fettsäurekette - (CH₂)m-COOH ist, wobei m 12 bis 19 ist.

9. Konjugat des IL-15-Analogons nach Anspruch 7, wobei das IL-15-Analogon und die Fettsäurekette durch *in* vitro-Kopplung verbunden sind.

10. Konjugat des IL-15-Analogons nach Anspruch 9, wobei die *in vitro*-Kopplung ein Verfahren umfasst, ausgewählt aus der Gruppe, bestehend aus:
1) Kopplung durch enzymatische Reaktion, wobei die Fettsäurekette in der enzymatischen Reaktion am N-Terminus GGG aufweist;
2) Kopplung mit freien Cysteinresten, die in das IL-15-Analogon eingeführt werden; und
3) Kopplung mit der Aminogruppe am N-Terminus des IL-15-Analogons.

11. Konjugat des IL-15-Analogons nach Anspruch 10, wobei
bei Kopplung durch enzymatische Reaktion die Fettsäurekette am Ende 3 Glycinreste aufweist;
bei Kopplung mit freien Cysteinresten, die in das IL-15-Analogon eingeführt werden, die Fettsäurekette einen Maleimidester oder eine halogenierte reaktive Gruppe aufweist; und
bei Kopplung mit der Aminogruppe am N-Terminus des IL-15-Analogons die Fettsäurekette eine Aldehydgruppe oder eine funktionelle Succinimidylestergruppe aufweist.

12. Konjugat des IL-15-Analogons nach Anspruch 7, wobei das IL-15-Analogon durch Anhängen einer -GS-LPETG umfassenden Sequenz an das Ende der Aminosäuresequenz von IL-15 erhalten wird.

13. Konjugat des IL-15-Analogons nach Anspruch 7, wobei die Fettsäurekette ausgewählt ist aus der Gruppe bestehend aus:
GGG-PEG2-Lys-(CH₂)₁₆-COOH,
NHS-PEG2-PEG2-γ-Glu-(CH₂)₁₇-COOH,
GGG-PEG4-PEG4-PEG4-Lys-(CH₂)₁₇-COOH,
GGG-PEG4-γ-Glu-γ-Glu-Lys-(CH₂)₁₇-COOH,
HOOC-(CH₂)₁₆-γ-Glu-γ-Glu-Lys-GGG,
HOOC-(CH₂)₁₆-CONH-γ-Glu-γ-Glu-PEG2-Lys-Br,
GGG-γ-Glu-C2DA-20EG-γ-Glu-(CH₂)₁₇-COOH, GGG-γ-Glu-C2DA-20EG-γ-Glu-(CH₂)₁₉-COOH, GGG-OEG-C2DA-20EG-γ-Glu-(CH₂)₁₉-COOH, GGG-OEG-C2DA-20EG-γ-Glu-Trx-(CH₂)₁₉-COOH,
CHO-PEG2-PEG2-γ-Glu-(CH₂)₁₇-COOH, und
Mal-C2DA-20EG-γ-Glu-Tn-(CH₂)₁₉-COOH.

## Revendications

1. Analogue d'IL-15, dans lequel la séquence d'acides aminés de l'analogue d'IL-15 comprend la séquence d'acides aminés d'IL-15 et un ou plusieurs acides aminés ajoutés à l'extrémité C-terminale de la séquence d'acides aminés d'iL-15,
les un ou plusieurs acides aminés ajoutés à l'extrémité C-terminale de la séquence d'acides aminés d'IL-15 étant choisis dans le groupe de :
GSGSGS-HHHHHH, GS-HHHHHH, PLASTKKR, LPKSAKKK, KKKKKKK, GAPQGAPQ-LVESAHHH, GS-LVSSAHHK, GS-LIEHHRRK, GS-IVEHRKKK, GS-VPKTGRRR, GS-LVASGKK, GS-HRKSGHHH, GS-LPKTGRHK, KKKTGRRH, LPRSGRHK, LVETHHHH, VRPETHHH, KKK, RHHHH, KRETHHHHH, GS-LPETG-GSGSHHHHHH, HLETGKKK, HVESGRRR, RRHTGKKK, HVKTGHHH, HVKSGRHH, HVKSSHRH, GSGSGSGSGS-LVKSGHHH, RPKSGHHK, KKC, LHKAGKHH, K et KK et la séquence d'acides aminés d'IL-15 étant montrée dans SEQ ID n° 1.

2. Séquence nucléotidique codant pour l'analogie d'IL-15 selon la revendication 1.

3. Procédé de préparation d'un analogue d'IL-15, dans lequel l'analogue d'IL-15 selon la revendication 1 est exprimé dans un système procaryote.

4. Procédé de préparation de l'analogue d'IL-15 selon la revendication 3, dans lequel une séquence nucléotidique codant pour l'analogue d'IL-15 est liée à un vecteur d'expression procaryote et transférée dans une bactérie hôte d'expression procaryote, qui est induite pour exprimer l'analogue d'IL-15.

5. Vecteur d'expression recombinant, comprenant une séquence nucléotidique codant pour l'analogue d'IL-15 selon la revendication 1.

6. Bactérie hôte transformée avec une séquence nucléotidique codant pour l'analogue d'IL-15 selon la revendication 1.

7. Conjugué d'un analogue d'IL-15, dans lequel la séquence d'acides aminés de l'analogue d'IL-15 comprend la séquence d'acides aminés d'IL-15 et un ou plusieurs acides aminés ajoutés à l'extrémité C-terminale de la séquence d'acides aminés d'IL-15 ; et le conjugué de l'analogue d'IL-15 comprenant l'analogue d'IL-15 selon la revendication 1 et une chaîne d'acide gras.

8. Conjugué de l'analogue d'IL-15 selon la revendication 7 dans lequel la chaîn d'acue gras est -(CH₂)ₘ-COOH, m valant 12 à 19.

9. Conjugué de l'analogue d'IL-15 selon la revendication 7 dans lequel l'analogue d'IL-15 et la chaîne d'acide gras sont couplés *in vitro.*

10. Conjugué de l'analogue d'IL-15 selon la revendication 9, dans lequel le couplage *in vitro* comprend un procédé choisi dans le groupe de :
1) couplage par réaction enzymatique, la chaîne d'acide gras dans la réaction enzymatique ayant GGG à l'extrémité N-terminale ;
2) couplage avec des résidus cystéine libres introduits dans l'analogue d'IL-15 ; et
3) couplage avec le groupe amino à l'extrémité N-terminale de l'analogue d'IL-15.

11. Conjugué de l'analogue d'IL-15 selon la revendication 10, dans lequel
quand le couplage s'effectue par réaction enzymatique, la chaîne d'acides gras a trois résidus glycine à l'extrémité terminale ;
quand le couplage s'effectue avec les résidus cystéine libres introduits dans l'analogue d'IL-15, la chaîne d'acide gras a un ester de maléimide ou un groupe réactif halogéné ; et
quand le couplage s'effectue avec le groupe amino à l'extrémité N-terminale de l'analogue d'IL-15, la chaîne d'acide gras a un groupe aldéhyde ou un groupe fonctionnel ester de succinimidyle.

12. Conjugué de l'analogue d'IL-15 selon la revendication 7, dans lequel l'analogue d'IL-15 est obtenu par addition d'une séquence comprenant -GS-LPETG à l'extrémité terminale de la séquence d'acides aminés d'IL-15.

13. Conjugué de l'analogue d'IL-15 selon la revendication 7, dans lequel la chaîne d'acide gras est choisie dans le groupe constitué par :
GGG-PEG2-Lys-(CH₂)₁₆-COOH,
NHS-PEG2-PEG2-γ-Glu-(CH₂)₁₇-COOH,
GGG-PEG4-PEG4-PEG4-Lys-(CH₂)₁₇-COOH,
GGG-PEG4-γ-Glu-γ-Glu-Lys-(CH₂)₁₇-COOH,
HOOC-(CH₂)₁₆-γ-Glu-γ-Glu-Lys-GGG,
HOOC-(CH₂)₁₆-CONH-γ-Glu-γ-Glu-PEG2-Lys-Br, GGG-γ-Glu-C2DA-2OEG-γ-Glu-(CH₂)₁₇-COOH, GGG-γ-Glu-C2DA-2OEG-y-Glu-(CH₂)₁₉-COOH, GGG-OEG-C2DA-2OEG-γ-Glu-(CH₂)₁₉-COOH, GGG-OEG-C2DA-2OEG-γ-Glu-Trx-(CH₂)₁₉-COOH,
CHO-PEG2-PEG2-γ-Glu-(CH₂)₁₇-COOH et
Mal-C2DA-2OEG-y-Glu-Tn-(CH₂)₁₉-COOH.
